# EUROPEAN PATENT SPECIFICATION

(11) **EP 2 932 925 B1**
(45) Date of publication and mention of the grant of the patent: **26.10.2016**
(21) Application number: 15169035.1
(22) Date of filing: 14.03.2014
(51) Int. Cl.: A61B 17/66

(54) **ORTHOPEDIC COMPRESSION/DISTRACTION DEVICE**
ORTHOPÄDISCHE KOMPRESSIONS-/DISTRAKTIONSVORRICHTUNG
DISPOSITIF DE DISTRACTION/COMPRESSION ORTHOPÉDIQUE

(30) Priority: 14.03.2013 US 201361782759 P
(43) Date of publication of application: 21.10.2015
(62) Divisional of application: 14768929.3
(73) Proprietor: Wright Medical Technology, Inc., Memphis, TN 38117 (US)
(72) Inventor: Thoren, Brian, Memphis, TN 38122 (US); McCormick, Daniel, Memphis, TN 38119 (US); Reed, Wesley, Libertyville, IL 60048 (US); Cramer, Thomas, Gainesville, FL 32605 (US); Lowery, Gary, Eads, TN 32028 (US); Harness, David, Eads, TN 38028 (US)
(74) Representative: Hirsch & Associés

(56) References cited:
- WO-A2-02/053038
- US-A1- 2006 247 649

## Description

### FIELD OF THE INVENTION

The present disclosure relates to an orthopedic device for compression or distraction of bone parts.

### BACKGROUND

Orthopedic devices utilizing elongated pins as fasteners for compression or distraction of bone parts finds many uses for treating orthopedic patients. "Elongated pins" will be used herein to refer to various pins and wires, such as K-wires, used for fixating bone parts or providing anchors. Therefore, there is a continuing need for an improved orthopedic device that expands the scope and ability of the orthopedic surgeons in treating patients in a variety of conditions.

US 2006/0247649 A1 discloses an assembly according to the preamble of claim 1.

### SUMMARY

The present invention provides an assembly as defined in claim 1 and an orthopedic device as defined in claim 2. Preferred embodiments are defined in the dependent claims.

### BRIEF DESCRIPTION OF THE DRAWINGS

**FIG. 1** **is** an illustration of an orthopedic device according to the present disclosure showing the locking sleeve attachments swiveled toward each other while the orthopedic device is in a distraction mode.
**FIG. 2A** is an illustration of the orthopedic device of FIG. 1 with the locking sleeve attachments removed.
**FIG. 2B** is an illustration showing the rack-and-pinion mechanism of the orthopedic device of FIG. 1.
**FIG. 3** is an illustration of and embodiment of the orthopedic device of FIG. 1 with 3-point bending yoke attachments.
**FIG. 4A** is an illustration of a biaxially swiveling locking sleeve attachment in its straight position viewed along its x-axis.
**FIG. 4B** is an illustration of the biaxially swiveling locking sleeve swiveled about the x-axis.
**FIG. 5A** is an illustration of the biaxially swiveling locking sleeve swiveled about its y-axis.
**FIG. 5B** is an illustration of the biaxially swiveling locking sleeve swiveled about the x-axis and the y-axis.
**FIG. 6A** is an illustration of the locking sleeve connected to the biaxial hinge block viewed along the *y*-axis showing the plane K-K extending through the center of the locking sleeve along which the cross-sectional view of FIG. 6B is taken.
**FIG. 6B** is a cross-sectional view along K-K taken through the biaxially swiveling locking sleeve attachment in its swiveling position.
**FIG. 7A** is the illustration of FIG. 6A showing the plane L-L extending off-center through the locking sleeve along which the cross-sectional view of FIG. 7B is taken.
**FIG. 7B** is a cross-sectional view along L-L taken through the biaxially swiveling locking sleeve attachment in its swiveling position.
**FIG. 8A** is the illustration of FIG. 6A showing the plane M-M extending through the center of the locking sleeve along which the cross-sectional view of FIG. 8B is taken.
**FIG. 8B** is a cross-sectional view along M-M taken through the biaxially swiveling locking sleeve attachment in its non-swiveling position.
**FIG. 9A** is the illustration of FIG. 6A showing the plane N-N extending off-center through the locking sleeve along which the cross-sectional view of FIG. 9B is taken.
**FIG. 9B** is a cross-sectional view along N-N taken through the biaxially swiveling locking sleeve attachment in its swiveling position.
**FIG. 10A** is an illustration of the biaxially swiveling locking sleeve in its non-swiveling position shown with only the y-axis locking pin portion of the biaxial hinge block shown.
**FIG. 10B** is an illustration of the biaxially swiveling locking sleeve in its swiveling position shown with only the y-axis locking pin portion of the biaxial hinge block shown.
**FIG. 11A** is an illustration of the biaxially swiveling locking sleeve in the same configuration as in FIG. 10A viewed from a different angle.
**FIG. 11B** is an illustration of the biaxially swiveling locking sleeve in the same configuration as in FIG. 10B viewed from a different angle.
**FIG. 12** is an exploded orthographic view showing the threaded collet end of a locking sleeve and a collet nut where the collet nut is shown in cross-section.

The features shown in the above referenced drawings are illustrated schematically and are not intended to be drawn to scale nor are they intended to be shown in precise positional relationship. Like reference numbers indicate like elements.

### DETAILED DESCRIPTION

This description of the exemplary embodiments is intended to be read in connection with the accompanying drawings, which are to be considered part of the entire written description. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

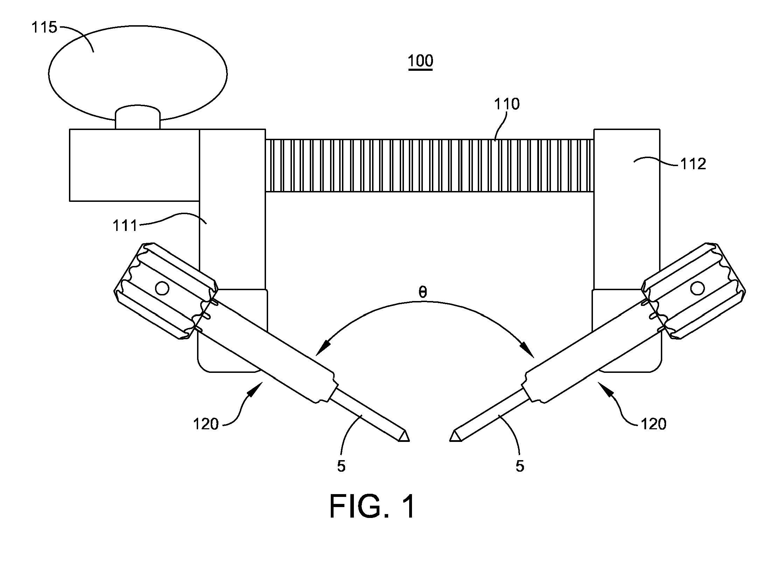

FIGS. 1-2B show an orthopedic device **100** that can be used for compression or distraction of bone parts according to an aspect of the present disclosure. The orthopedic device **100** comprises an elongated body **110** having first end **10** and a second end **20.** A first arm member **112** extends away from the first end **10** in a direction transverse to the elongated body **110.** The first arm member **112** can be integrally formed with the body **110** or otherwise attached to the elongated body **110.** A second arm member **111** transversely extends away from the elongated body **110.** The second arm member **111** is configured with a base portion **113** that is configured and adapted to movably engage the elongated body **110** allowing the second arm member **111** to be longitudinally movable along the elongated body to accomplish compression or distraction function of the device.

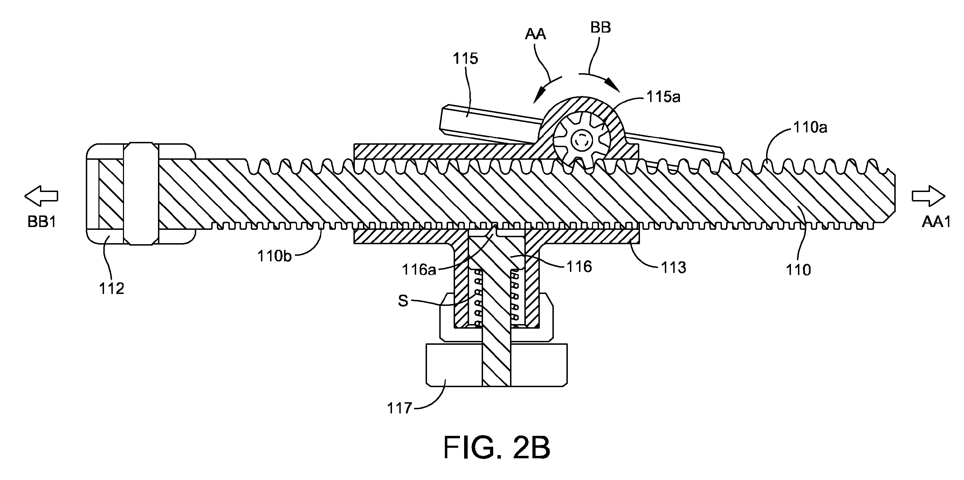

The actual structural mechanism for enabling the movable engagement between the elongated body **110** and the second arm member **111** can be one of many such structures known in the art. Referring to FIG. 2B, in one embodiment, the elongated body **110** is a rack of gear teeth **110a** and a pinion gear **115a** provided on the base portion **113** engages the rack of gear teeth **110a** for longitudinally moving the second arm member **111** along the elongated body. The pinion gear **115a** is rotatably attached to the base portion **113** and the second arm member **111** is moved longitudinally by turning the pinion gear **115a.** The pinion gear **115a** is provided with a bow **115,** similar to a bow on a key, to enable a user to turn the pinion gear.

The base portion **113** is configured with ratcheting mechanisms that can selectably operate in compression or distraction mode. For example, the base portion **113** can be provided with a spring-loaded ratcheting pin mechanism **116** for limiting the moving direction of the second arm member **111** along the elongated body **110** to be a one-way movement, as shown in FIG. 2B.

In the configuration shown in FIG. 2B, the spring-loaded ratcheting pin mechanism **116** is arranged so that the ratcheting pin mechanism **116** engages the ratchet teeth **110b** by a detent **116a.** The coil spring **S** urges the detent **116a** against the ratchet teeth **110b.** The detent **116a** has a slanted surface on one side as shown so that the ratchet teeth **110b** can depress the detent **116a** against the spring S and, thus, gliding over the detent **116a.** The elongated body **110** can move in the direction of arrow **AA1** by turning the pinion gear **115a** in the direction of the arrow **AA**. The detent **116a** will prevent the elongated body **110** from moving in the opposite direction shown by the arrow **BB1.**

In order to move the elongated body **110** in the direction of the arrow **BB1,** the spring-loaded ratcheting pin mechanism **116** is turned 180 degrees so that the slanted side of the detent **116a** is now facing in the opposite direction. This feature is used to change the direction of the one-way movement second arm member **111** so that the operation of the orthopedic device **100** is changed from compression to distraction and vice versa. The spring loaded ratcheting pin mechanism **116** can be turned 180 degrees by pulling the pin mechanism out using the thumb wheel **117,** turning it 180 degrees and releasing it. The spring bias will return the pin mechanism **116** to the seated position but with the detent **116a** now facing 180 degrees from before. Then, the pinion gear **115a** can be turned in the direction of the arrow **BB** and move the elongated body **110** in the direction of the arrow **BB1.** Another example of the ratcheting mechanism between the base portion **113** and the elongated body **110** is described in United States patent Application Serial No. 13/712,300, filed by the Applicant on December 12, 2012,

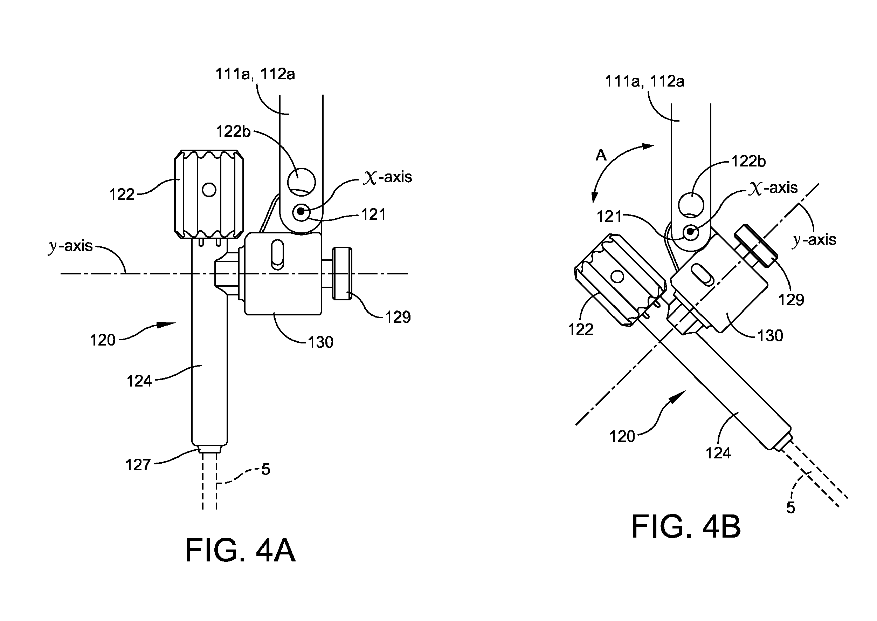

In the orthopedic device **100** according to the present disclosure shown I FIG. 1, locking sleeve **120** attachments are hingeably connected to the outer ends of the first and second arm members **111, 112** by a biaxial hinge block **130** shown in FIGs. 4A-9B. The locking sleeves **120** are configured for receiving and locking on to an elongated pin and the biaxial hinge block **130** is configured to allow each of the locking sleeve to swivel in two different directions with respect to its corresponding arm member, **111** or **112,** about two orthogonally oriented axes. The biaxial hinge block **130** is shown and described in detail below in conjunction with FIGS. 4A through 9B.

In FIG. 1, the orthopedic device **100** is in distraction mode and the locking sleeve attachments **120** holding elongated pins 5 are shown swiveled toward each other forming an angle θ between the two locking sleeve attachments **120.**

Referring to FIGS. 4A through 5C, the orthopedic device **100** also includes a locking sleeve **120** hingeably connected to the outer end of each of the first and second arm members **111, 112** by a biaxial hinge block **130,** wherein each locking sleeve **120** is configured for lockably receiving an elongated pin (not shown).

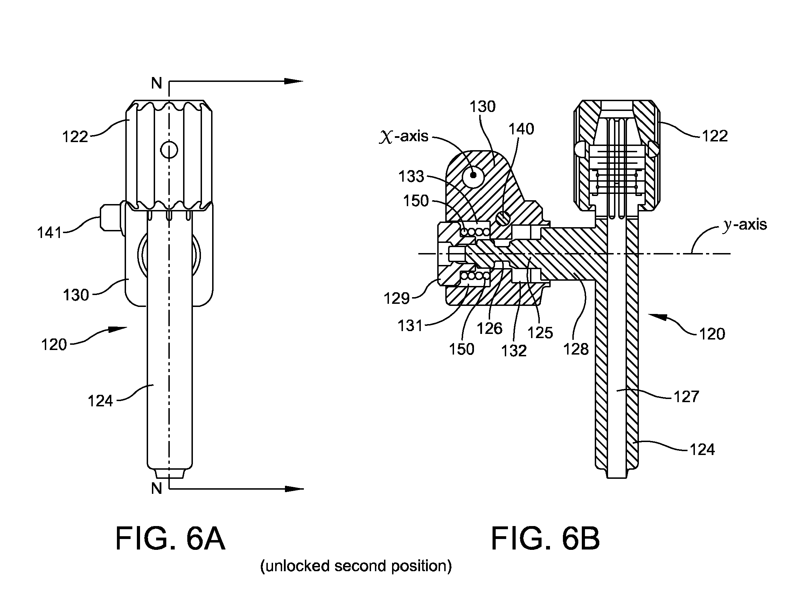

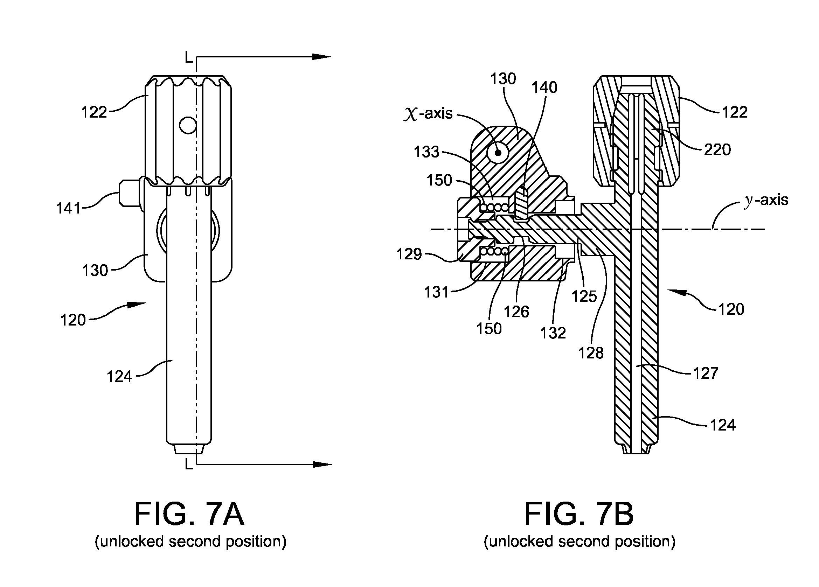

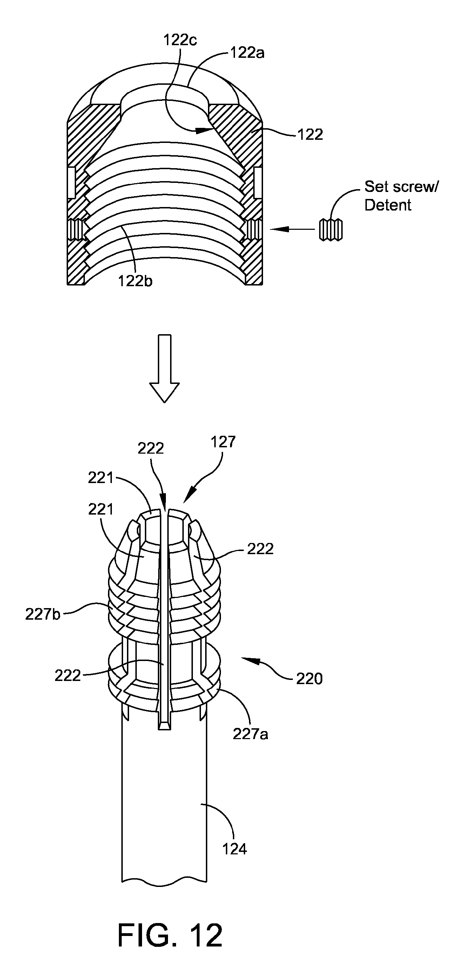

The locking sleeve **120** comprises an elongated shaft **124** having an elongated pin receiving bore **127** (see FIGS. 6B, 7B, 8B, 9B) extending therethrough and a threaded collet **220** (see FIG. 7) provided at one end of the elongated shaft for locking onto or securely holding the elongated pin received in the pin receiving bore **127.** Referring to FIG. 12, the threaded collet **220** comprises a plurality of collet arms **221,** defined by slots **222,** provided with screw threads **227a, 227b** integrally formed on their exterior surfaces, and a collet nut **122** that is threadably engaged to the threaded collet **220** for locking or securely holding the elongated pin **5** that is received in the elongated pin receiving bore **127.**

FIG. 12 shows detailed structures of the threaded collet **220** and the collet nut **122.** As shown in the longitudinal cross-section view of the collet nut **122** in FIG. 12, the collet nut **122** is open at one end for receiving the threaded collet **220** and has an interior surface provided with screw threads **122b** for threadably engaging the screw threads **227a, 227b** of the collet **220.** At the end opposite from the threaded collet receiving end, a through hole **122a** is provided for the elongated pin received in the elongated pin receiving bore **127.** The interior surface of the collet nut **122** is configured with a conical surface **122c** for engaging collet arms **221.** When an elongated pin **5** is received in the elongated pin receiving bore **127,** the threading action of the collet nut **122** causes the collet arms **221** to move radially inward and clamp onto the elongated pin and lock the elongated pin in place. Generally, it is envisioned that the user can lock an elongated pin received in the elongated receiving bore **127** by tightening the collet nut **122** by hand. Further details of the structures of the threaded collet **220** and the collet nut **122** can be found in the United States patent Application Serial No. 13/712,300, filed by the Applicant on December 12, 2012.

As shown in FIGS. 4A through 5C, each of the biaxial hinge blocks **130** is configured to allow the locking sleeve **120** to swivel in two directions about two orthogonally oriented swivel axes, *x*-axis and *y*-axis. The biaxial hinge blocks **130** can connect the locking sleeves **120** directly to the outer ends of one of the arm members **111, 112** similar to the way the hinge joints in the orthopedic device disclosed in United States patent Application Serial No. 13/712,300 connect the locking sleeves to the outer ends of the arm members **111, 112.** In the embodiment of the present disclosure, the outer ends of the arm members **111, 112** are configured for modular connection of the locking sleeves **120** or some other attachments.

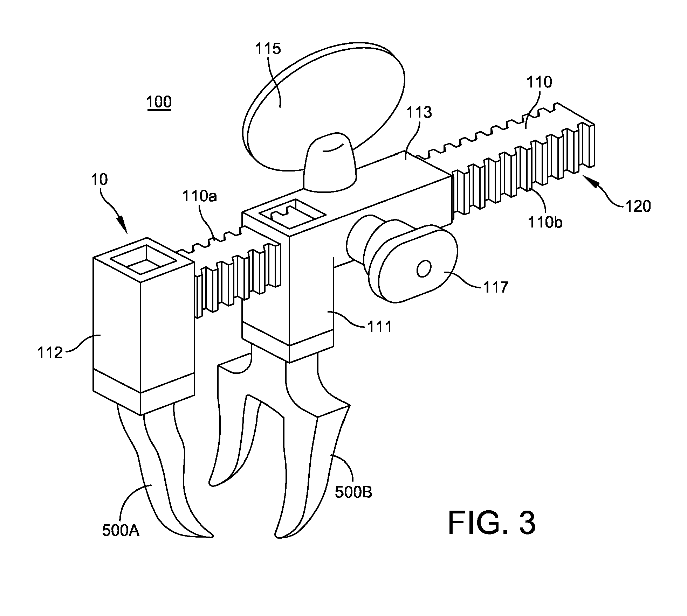

An example of the orthopedic device **100** with 3-point bending yoke attachments **500A** and **500B** attached to the first and second arm members **111, 112** is shown in FIG. 3. Such 3-point bending yoke attachments can be used to accomplish radial distraction of a bone segment.

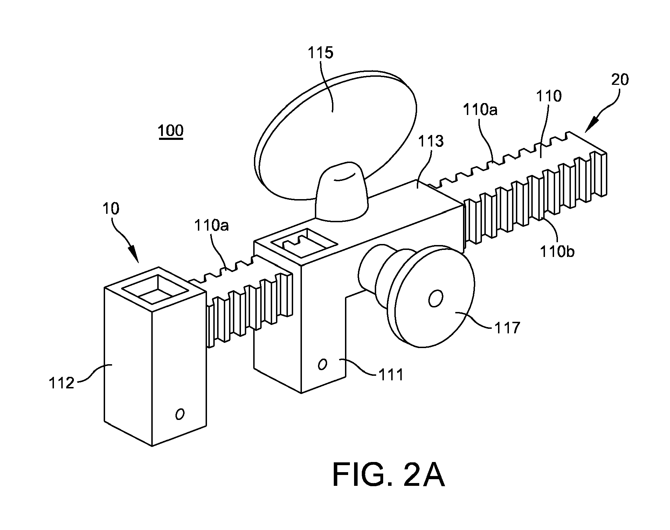

As shown in FIG. 2A, the outer ends of the first and second arm members **111, 112** are configured to receive attachments. As shown in FIG. 5C, the biaxial hinge block **130** is hingeably connected to an arm extension piece **111a, 112a** which is configured to removably attach to the first and second arm members **111, 112.** The specific structures that will enable such attachment between the first and second arm members and the arm extension pieces can be one of a variety of structures that are well known or obvious to one of ordinary skill in the art and need not be described in detail here.

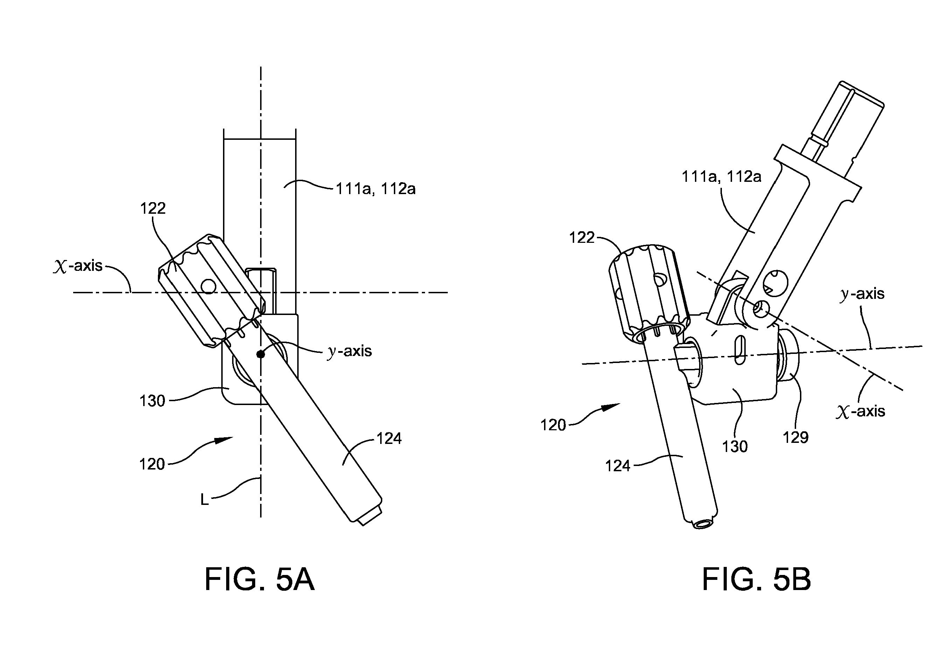

Referring to FIGS. 4A and 4B, the biaxial hinge block **130** includes a first hinge joint that swivels about a first swivel axis, *x*-axis. The first hinge joint allows the locking sleeve **120** to swivel about the *x*-axis in a first direction which is represented by the arrow **A** in FIG. 4B. The first hinge joint can include a swivel pin **121** forming the *x-*axis and connecting the biaxial hinge block **130** to the arm extension piece **111a, 112a.** The biaxial hinge block **130** also includes a second hinge joint that swivels about a second swivel axis, *y*-axis, that is orthogonal to the first swivel axis. Thus, the biaxial hinge block **130** allows the locking sleeve **120** to swivel in two directions about the two orthogonally oriented swivel axes. FIG. 5A shows the locking sleeve **120** swiveled about the second swivel axis, *y*-axis, so that the locking sleeve **120** is tilted away from the longitudinal axis **L** of the arm extension pieces **111a, 112a.** FIG. 5B shows an orthogonal projection view of the locking sleeve **120** swiveled about both swivel axes of the biaxial hinge block **130.**

In the illustrated example, the first hinge joint is formed by a pin 20 that is aligned with the *x*-axis and connects the biaxial hinge block **130** to the arm extension pieces **111a, 112a.** The first swivel axis, *x*-axis, is oriented parallel to the elongated body **110** of the orthopedic device.

According to an aspect of the present disclosure, the first hinge joint can be configured and adapted to be normally locked at a desired swivel angle and prevented from swiveling about the first swivel axis, *x*-axis, by a spring-loaded locking pin **112b.** When the spring-loaded locking pin **112b** is pressed, the first hinge joint is unlocked and free to swivel about the *x*-axis.

The second hinge joint of the biaxial hinge block **130** will be described in more detail using the additional FIGS. 6A through 11B. The second hinge joint is formed by a swiveling shaft **125,** that is orthogonally extending from the elongated shaft of the locking sleeve **120** and received in the biaxial hinge block **130** along the second swivel axis, *y*-axis. The swiveling shaft **125** is movable within the biaxial hinge block **130** along the second swivel axis between two positions. A locked first position that keeps the locking sleeve **120** in a non-swiveling position preventing the locking sleeve **120** from swiveling about the second swivel axis, and an unlocked second position allowing the locking sleeve **120** to swivel about the second swivel axis.

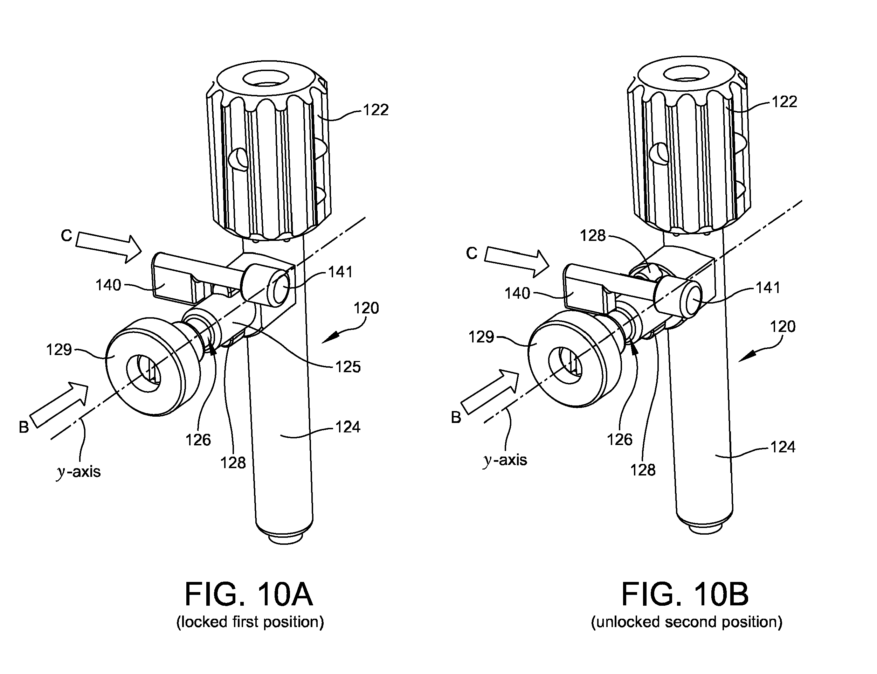

FIGS. 6A - 7B show the swiveling shaft **125** in its unlocked second position within the biaxial hinge block **130,** wherein the locking sleeve attachment **120** can swivel about the *y*-axis. FIG. 6A is an illustration of the locking sleeve attachment **120** connected to the biaxial hinge block **130** viewed along the *y*-axis showing the plane K-K extending through the center of the locking sleeve attachment **120** along which the cross-sectional view of FIG. 6B is taken. FIG. 6B is the cross-sectional view taken along K-K. FIG. 7A is the illustration of FIG. 6A showing the plane L-L extending off-center through the locking sleeve attachment **120** along which the cross-sectional view of FIG. 7B is taken. FIG. 7B is a cross-sectional view along L-L. The biaxial hinge block **130** is provided with a through-hole **131** for receiving the swiveling shaft **125** therethrough. The swiveling shaft **125** has an annular groove **126** and the biaxial hinge block **130** is provided with a locking key **140** that engages the annular groove **126** and keeps the locking sleeve **120** in the unlocked second position. The engaging relationship between the locking key **140** and the swiveling shaft **125** can be better seen in FIG. 10B in which only the locking sleeve's swiveling shaft **125** and the locking key **140** are shown without the biaxial hinge block **130.** The locking key **140** sits within the annular groove **126** and prevents the swiveling shaft **125** from moving along the *y*-axis while allowing the swiveling shaft **125** and, thus, the locking sleeve **120** to swivel about the *y*-axis.

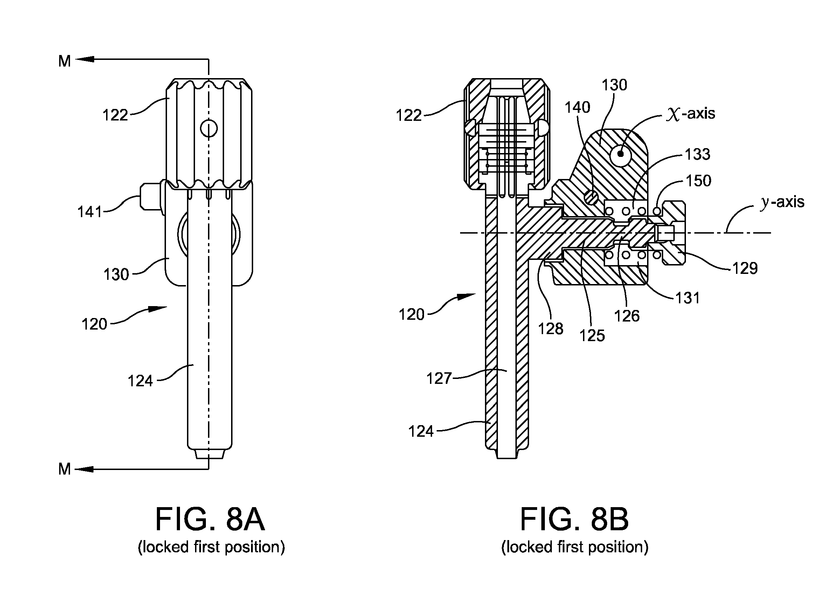

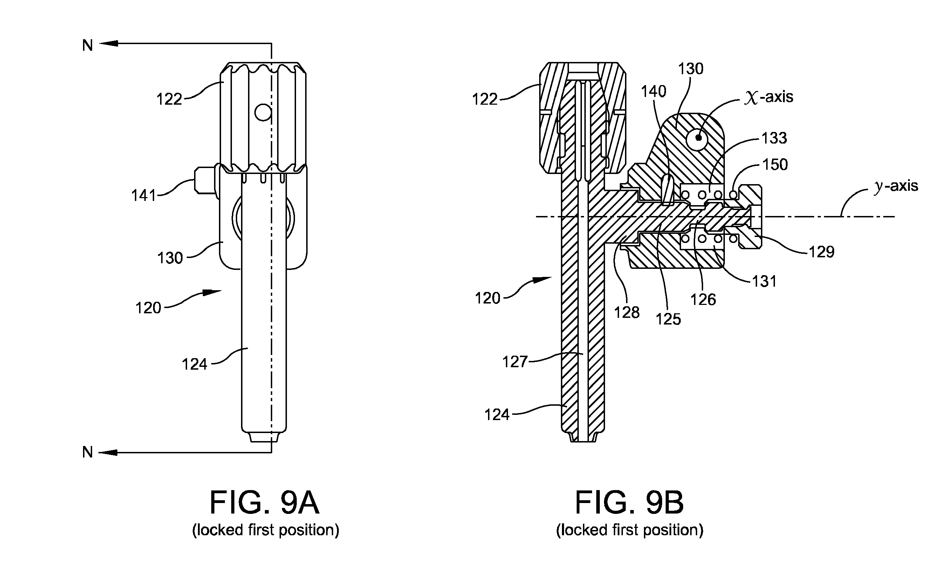

FIGS. 8A - 9B show the swiveling shaft **125** in its locked first position, the non-swiveling position, within the biaxial hinge block **130.** The locking sleeve attachment **120** is in a fixed orientation and cannot swivel about the *y*-axis. FIG. 8A is the same view as FIG. 6A but showing the plane M-M extending through the center of the locking sleeve along which the cross-sectional view of FIG. 8B is taken. FIG. 8B is a cross-sectional view taken along M-M. FIG. 9A is the same view as FIG. 6A but showing the plane N-N extending off-center through the locking sleeve along which the cross-sectional view of FIG. 9B is taken. FIG. 9B is a cross-sectional view taken along N-N. In the locked first position, the swiveling shaft **125** is pushed further into the through-hole **131** of the biaxial hinge block **130** so that the locking key **140** is no longer sitting within the annular groove **126.** Referring to FIGS. 10A - 11B, the swiveling shaft **125** is provided with one or more alignment tabs **128** near the base portion (the part of the elongated shaft **124** connected to the elongated shaft **124)** of the swiveling shaft **125** and the through-hole **131** of the biaxial hinge block **130** has an alignment-tab-receiving end **132** (see FIG. 7B) that is configured and adapted to receive the alignment tabs **128.** Preferably, the alignment tabs **128** are two tabs oppositely located on the base portion of the swiveling shaft but other non-symmetrically positioned arrangements are also contemplated.

The alignment-tab-receiving end **132** of the through-hole **131** has an opening outline that matches the transverse cross-sectional outline of the alignment tabs **128** so that in the locked first position, where the swiveling shaft **125** is pushed further into the through-hole **131,** the alignment tabs **128** engage or mate with the alignment-tab-receiving end **132** and prevent the locking sleeve from swiveling about the *y*-axis.

In one embodiment, the locked first position holds the locking sleeve **120** in an orientation that keeps the elongated shaft **124** of the locking sleeve **120** in a parallel orientation with the arm members **111, 112** of the orthopedic device. In another embodiment, the alignment tabs **128** and the alignment-tab-receiving end **132** can be configured to hold the locking sleeve **120** in any desired angular orientation about the *y-*axis.

Referring to FIGS. 6A-9B, the swiveling shaft **125** is provided with an end cap **129** that captures a coil spring **150** inside an end-cap-receiving portion **133** of the through-hole **131.** The coil spring is in a normally compressed state inside the end-cap-receiving portion **133** so that the coil spring is always urging against the end cap **129** pulling the swiveling shaft **125** further into the through-hole **131** and toward the locked first position shown in FIGS. 8A - 9B.

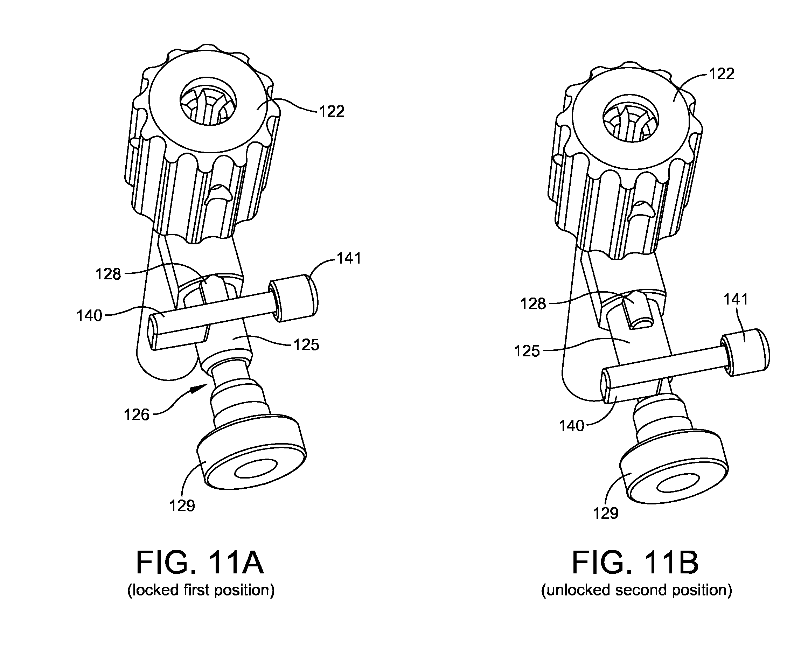

FIGS. 10A and 11A show the positional relationship of the locking key **140** and the swiveling shaft **125** when the swiveling shaft **125** is in the locked first position. In the locked first position, the locking key **140** is not sitting in the annular groove **126.**

According to an embodiment, the locking key **140** is spring-loaded within the biaxial hinge block **130** to be urged in the direction **C** shown in FIGS. 10A and 10B, which is transverse to the *y*-axis, the second swivel axis. To unlock the swiveling shaft **125** from the locked first position shown in FIGS. 9B and 10A, the end cap **129** of the swiveling shaft is pushed in the direction **B** shown in FIG. 10A. This moves the swiveling shaft **125** along the y-axis into its unlocked second position and the annular groove **126** comes in alignment with the locking key **140.** Because the locking key **140** is spring-loaded and being urged in the direction C, the locking key **140** will slide into the annular groove **126** and prevents the swiveling shaft **125** from backing out and keeps the swiveling shaft **125** in its unlocked second position. To move the swiveling shaft **125** back to its non-swiveling first position, the end cap **141** of the locking key **140** is pushed in the direction opposite **C,** thus sliding the locking key **140** out of the annular groove **126.** With the locking key **140** out of the annular groove **126,** the spring-loaded swiveling shaft **125** will slide along the y-axis in direct opposite **B** and return to its first position if he alignment tabs **128** are aligned with the mating outline of the alignment-tab-receiving end **132** of the through-hole **131.**

As described, the orthopedic device **100** of the present disclosure is a universal device that can be used for compression or distraction of bone parts that are secured to the first and second arm members **112, 111** by elongated pins, such as K-wires, locked into the elongated pin receiving bores **127** of the locking sleeves **120.** Referring to FIG. 1, after the bone parts are secured, the movable second arm member **111** can be moved in compression direction **201** or distraction direction **202** by turning the turning key handle **115.**

Although the invention has been described in terms of exemplary embodiments, it is not limited thereto. Rather, the appended claims should be construed broadly, to include other variants and embodiments of the invention, which may be made by those skilled in the art without departing from the scope and range of equivalents of the invention. The scope of the invention disclosed herein is to be limited only by the following claims.

## Claims

1. An assembly comprising:
a biaxial hinge block (130); and
a locking sleeve (120) comprising an elongated shaft (124) having an elongated pin receiving bore (127) extending therethrough wherein the biaxial hinge block and the locking sleeve are configured to hingeably connect the locking sleeve to an arm member of an orthopedic device, said biaxial hinge block being configured to allow the locking sleeve to swivel in two different directions with respect to the arm member about two orthogonally oriented swivel axes (x-axis and y-axis),
said locking sleeve is provided with a swiveling shaft (125) orthogonally extending from the elongated shaft (124), said locking sleeve connected to the biaxial hinge block by the swiveling shaft being received in the biaxial hinge block along the second swivel axis (y-axis),
**characterized in that**
the swiveling shaft (125) is movable within the biaxial hinge block (130) along the second swivel axis (y-axis) between two positions, a first position that prevents the locking sleeve from swiveling about the second swivel axis and a second position allowing the locking sleeve to swivel about the second swivel axis.

2. An orthopedic device comprising
two assemblies of claim 1;
an elongated body having first and second ends;
a first arm member attached to and extending away from said first end and terminating at an outer end;
a second arm member extending from said elongated body and having a base portion and an outer end, wherein the base portion is configured and adapted to engage the elongated body and allow the second arm member to be longitudinally movable along said elongated body, said second arm member extending from the elongated body in the same direction as the first arm member;
wherein one of the two locking sleeves is hingeably connected to the outer end of the first arm member by one of the two biaxial hinge blocks,
wherein the other locking sleeve is hingeably connected to the outer end of the second arm member by the other biaxial hinge block,
wherein the two locking sleeves are configured for receiving and locking on to two elongated pins, respectively.

3. The orthopedic device of claim 2, herein the first of the two orthogonally oriented swivel axes is oriented parallel to the elongated body of the orthopedic device.

4. The assembly of claim 1, wherein said locking sleeve further comprises a collet (220) provided at one end of the elongated shaft, said collet comprising a plurality of collet arms (221) provided with screw threads (227a, 227b) integrally formed on their exterior surfaces, and a collet nut (122) that is threadably engaged to the collet for locking an elongated pin (5) that is received in the elongated pin receiving bore.

5. The assembly of claim 1, wherein the biaxial hinge block is provided with a through-hole (131) for receiving the swiveling shaft therethrough, the through-hole having an end-cap-receiving portion (133), wherein the swiveling shaft is provided with an end cap (129) that captures a coil spring (150) inside the end-cap-receiving portion of the through-hole, wherein the coil spring is in a normally compressed state inside the end-cap-receiving portion urging against the end cap and pulling the swiveling shaft further into the through-hole toward the first position.

6. The assembly of claim 1, wherein the swiveling shaft is provided with one or more alignment tabs (128) near its base portion and the through-hole has an alignment-tab-receiving end that is configured and adapted to receive the alignment tabs, wherein the alignment-tab-receiving end of the through-hole has an opening outline that matches transverse cross-sectional outline of the alignment tabs thereby when in the first position, the alignment tabs engage with the alignment-tab-receiving end and prevent the locking sleeve from swiveling about the second swivel axis.

7. The assembly of claim 1, wherein the first position holds the locking sleeve in an orientation that keeps the elongated shaft of the locking sleeve in a parallel orientation with the arm members of the orthopedic device.

8. The assembly of claim 1, further comprising a locking key (140) provided in the biaxial hinge block that engages the swiveling shaft to lock the swiveling shaft in the second position.

9. The assembly of claim 8, wherein the locking key is spring-loaded within the biaxial hinge block to be urged in the direction transverse to the second swivel axis.

10. The assembly of claim 8, wherein the swiveling shaft is configured with an annular groove (126) that engages the locking key when the swiveling shaft is in the second position.

11. The assembly of claim 10, wherein when the swiveling shaft is in the first position, the locking key and the annular groove are not engaged while the alignment tabs provided on the swiveling shaft and the alignment-tab-receiving end are engaged and thus preventing the swiveling shaft from swiveling in the second swivel axis.

## Patentansprüche

1. Baugruppe, umfassend:
- einen zweiachsigen Scharnierblock (130); und
- eine Spannhülse (120), die eine gestreckte Spindel (124) umfasst, welche eine gestreckte stiftaufnehmende Bohrung (127) hat, die sich durch dieselbe erstreckt, wobei der zweiachsige Scharnierblock und die Spannhülse so ausgelegt sind, dass sie schwenkbar die Spannhülse mit einem Armelement einer orthopädischen Vorrichtung verbinden, wobei der zweiachsige Scharnierblock dafür ausgelegt ist, der Spannhülse zu ermöglichen, sich in zwei verschiedenen Richtungen gegenüber dem Armelement um zwei orthogonal ausgerichtete Schwenkachsen (x-Achse und y-Achse) zu drehen,
- die Spannhülse mit einer Schwenkspindel (125) versehen ist, die sich orthogonal von der gestreckten Spindel (124) aus erstreckt, wobei die Spannhülse, die mit dem zweiachsigen Scharnierblock durch die Schwenkspindel verbunden ist, im zweiachsigen Scharnierblock entlang der zweiten Schwenkachse (y-Achse) aufgenommen wird,
**dadurch gekennzeichnet, dass**
die Schwenkspindel (125) innerhalb des zweiachsigen Scharnierblocks (130) entlang der zweiten Schwenkachse (y-Achse) zwischen zwei Positionen beweglich ist, einer ersten Position, die die Spannhülse am Drehen um die zweite Schwenkachse hindert, und einer zweiten Position, die der Spannhülse ermöglicht, sich um die zweite Schwenkachse zu drehen.

2. Orthopädische Vorrichtung, umfassend:
- zwei Baugruppen nach Anspruch 1;
- einen gestreckten Körper, der ein erstes und ein zweites Ende hat;
- ein erstes Armelement, das am ersten Ende befestigt und sich weg von demselben erstreckt und an einem äußeren Ende endet;
- ein zweites Armelement, das sich vom gestreckten Körper aus erstreckt und einen Basisteil und ein äußeres Ende hat,
wobei der Basisteil dafür konfiguriert und ausgelegt ist,in den gestreckten Körper einzugreifen und dem zweiten Armelement zu ermöglichen, sich longitudinal entlang des gestreckten Körpers zu bewegen, wobei das zweite Armelement sich vom gestreckten Körper aus in derselben Richtung wie das erste Armelement erstreckt;
wobei eine der Spannhülsen schwenkbar mit dem äußeren Ende des ersten Armelementes durch eine der zwei zweiachsigen Scharnierblöcke verbunden ist,
wobei die andere Spannhülse schwenkbar mit dem äußeren Ende des zweiten Armelementes durch den anderen zweiachsigen Scharnierblock verbunden ist,
wobei die zwei Spannhülsen zum Aufnehmen bzw. Einrasten an zwei gestreckten Zapfen ausgelegt sind.

3. Orthopädische Vorrichtung nach Anspruch 2, wobei die erste der zwei orthogonal ausgerichteten Schwenkachsen parallel zum gestreckten Körper der orthopädischen Vorrichtung ausgerichtet ist.

4. Baugruppe nach Anspruch 1, wobei die Spannhülse ferner umfassteine Klemmhülse (220), die an einem Ende der gestreckten Spindel vorgesehen ist, wobei die Klemmhülse mehrere Klemmhülsenarme (221) umfasst, die mit Schraubgewinden (227a, 227b) versehen ist, die auf ihren äußeren Fläche integral geformt sind, und eine Klemmhülsennut (122), die schraubbar in die Klemmhülse zum Verriegeln eines gestreckten Stifts (5) eingreift, der in der gestreckten stiftaufnehmenden Bohrung aufgenommen ist.

5. Baugruppe nach Anspruch 1, wobei der zweiachsige Scharnierblock mit einem Durchgangsloch (131) zum Aufnehmen der Schwenkspindel in derselben vorgesehen ist, wobei das Durchgangsloch einen Endkappen aufnehmenden Teil (133) hat, wobei die Schwenkspindel mit einer Endkappe (129) versehen ist, die eine Schraubenfeder (150) innerhalb des Endkappen aufnehmenden Teils des Durchgangslochs erfasst , wobei die Schraubenfeder in einem normalerweise komprimierten Zustand innerhalb des Endkappen aufnehmenden Teils ist, der gegen die Endkappe drückt und die Schwenkspindel weiter in das Durchgangsloch zur ersten Position zieht.

6. Baugruppe nach Anspruch 1, wobei die Schwenkspindel mit einer oder mehreren Ausrichtungszungen (128) in der Nähe ihres Basisteils versehen ist, und das Durchgangsloch ein Ausrichtungszungen aufnehmendes Ende hat, das dafür konfiguriert und ausgelegt ist, die Ausrichtungszungen aufzunehmen, wobei das Ausrichtungszungen aufnehmende Ende des Durchgangslochs einen Öffnungsumriss hat, der zur querlaufenden Querschnittskontur der Ausrichtungszungen passt, wodurch in der ersten Position die Ausrichtungszungen in das Ausrichtungszungen aufnehmende Ende eingreifen und die Spannhülse daran hindern, sich um die zweite Schwenkachse zu drehen.

7. Baugruppe nach Anspruch 1, wobei die erste Position die Spannhülse in einer Orientierung hält, die die gestreckte Spindel der Spannhülse in einer parallelen Orientierung zu den Armelementen der orthopädischen Vorrichtung hält.

8. Baugruppe nach Anspruch 1, die ferner einen Arretiernocken (140) umfasst, der im zweiachsigen Scharnierblock vorgesehen ist, welcher in die Schwenkspindel eingreift, um die Schwenkspindel in der zweiten Position zu verriegeln.

9. Baugruppe nach Anspruch 8, wobei der Arretiernocken innerhalb des zweiachsigen Scharnierblocks federbelastet ist, damit er in der Richtung quer zur zweiten Schwenkachse gedrängt wird.

10. Baugruppe nach Anspruch 8, wobei die Schwenkspindel mit einer ringförmigen Nut (126) ausgelegt ist, die den Arretiernocken einrasten lässt, wenn die Schwenkspindel in der zweiten Position ist.

11. Baugruppe nach Anspruch 10, wobei, wenn die Schwenkspindel in der ersten Position ist, der Arretiernocken und die ringförmige Not nicht im Eingriff sind, während die Ausrichtungszungen, die auf der Schwenkspindel vorgesehen sind, und das Ausrichtungszungen aufnehmende Ende im Eingriff sind und so die Schwenkspindel daran hindern, sich um die zweite Schwenkachse zu drehen.

## Revendications

1. Ensemble comprenant :
un bloc de charnière biaxial (130) ; et
un manchon de blocage (120) comprenant un arbre allongé (124) ayant un alésage de réception de broche (127) s'étendant à travers ce dernier, dans lequel le bloc de charnière biaxial et le manchon de blocage sont configurés pour raccorder, par charnière, le manchon de blocage à un élément de bras d'un dispositif orthopédique, ledit bloc de charnière biaxial étant configuré pour permettre au manchon de blocage de pivoter dans deux directions différentes par rapport à l'élément de bras autour de deux axes de pivot orientés de manière orthogonale (axe x et axe y),
ledit manchon de blocage est prévu avec un arbre de pivotement (125) s'étendant de manière orthogonale à partir de l'arbre allongé (124), ledit manchon de blocage étant raccordé au bloc de charnière biaxial par l'arbre de pivotement qui est reçu dans le bloc de charnière biaxial le long du second axe de pivot (axe y),
**caractérisé en ce que** :
l'arbre de pivotement (125) est mobile à l'intérieur du bloc de charnière biaxial (130) le long du second axe de pivot (axe y) entre deux positions, une première position qui empêche le manchon de blocage de pivoter autour du second axe de pivot et une seconde position permettant au manchon de blocage de pivoter autour du second axe de pivot.

2. Dispositif orthopédique comprenant :
deux ensembles selon la revendication 1 ;
un corps allongé ayant des première et seconde extrémités ;
un premier élément de bras fixé sur et s'étendant à distance de ladite première extrémité et se terminant par une extrémité externe ;
un second élément de bras s'étendant à partir dudit corps allongé et ayant une partie de base et une extrémité externe, dans lequel la partie de base est configurée et adaptée pour mettre en prise le corps allongé et permettre au second élément de bras d'être longitudinalement mobile le long dudit corps allongé, ledit second élément de bras s'étendant à partir du corps allongé dans la même direction que le premier élément de bras ;
dans lequel l'un des deux manchons de blocage est raccordé, par charnière, à l'extrémité externe du premier élément de bras par l'un des deux blocs de charnière biaxiaux,
dans lequel l'autre manchon de blocage est raccordé, par charnière, à l'extrémité externe du second élément de bras par l'autre bloc de charnière biaxial,
dans lequel les deux manchons de blocage sont configurés pour recevoir et se bloquer sur deux broches allongées, respectivement.

3. Dispositif orthopédique selon la revendication 2, dans lequel le premier des deux axes de pivot orientés de manière orthogonale est orienté parallèlement au corps allongé du dispositif orthopédique.

4. Ensemble selon la revendication 1, dans lequel ledit manchon de blocage comprend en outre :
une bague de serrage (220) prévue au niveau d'une extrémité de l'arbre allongé, ladite bague de serrage comprenant une pluralité de bras de bague de serrage (221) prévus avec des filetages de vis (227a, 227b) formés de manière intégrale sur leurs surfaces extérieures, et un écrou de bague de serrage (122) qui est mis en prise par filetage sur la bague de serrage pour bloquer une broche allongée (5) qui est reçue dans l'alésage de réception de broche allongée.

5. Ensemble selon la revendication 1, dans lequel le bloc de charnière biaxial est prévu avec un trou débouchant (131) pour recevoir l'arbre de pivotement à travers ce dernier, le trou débouchant ayant une partie de réception de capuchon d'extrémité (133), dans lequel l'arbre de pivotement est prévu avec un capuchon d'extrémité (129) qui capture un ressort hélicoïdal (150) à l'intérieur de la partie de réception de capuchon d'extrémité du trou débouchant, dans lequel le ressort hélicoïdal est dans un état normalement comprimé à l'intérieur de la partie de réception de capuchon d'extrémité poussant contre le capuchon d'extrémité et tirant l'arbre de pivotement davantage dans le trou débouchant vers la première position.

6. Ensemble selon la revendication 1, dans lequel l'arbre de pivotement est prévu avec une ou plusieurs languettes d'alignement (128) à proximité de sa partie de base et le trou débouchant a une extrémité de réception de languette d'alignement qui est configurée et adaptée pour recevoir les languettes d'alignement, dans lequel l'extrémité de réception de languette d'alignement du trou débouchant a un contour d'ouverture qui correspond au contour transversal des languettes d'alignement ainsi, lorsqu'elles sont dans la première position, les languettes d'alignement se mettent en prise avec l'extrémité de réception de languette d'alignement et empêchent le manchon de blocage de pivoter autour du second axe de pivot.

7. Ensemble selon la revendication 1, dans lequel la première position maintient le manchon de blocage dans une orientation qui garde l'arbre allongé du manchon de blocage dans une orientation parallèle aux éléments de bras du dispositif orthopédique.

8. Ensemble selon la revendication 1, comprenant en outre une clavette de blocage (140) prévue dans le bloc de charnière biaxial qui met en prise l'arbre de pivotement pour bloquer l'arbre de pivotement dans la seconde position.

9. Ensemble selon la revendication 8, dans lequel la clavette de blocage est chargée par ressort à l'intérieur du bloc de charnière biaxial pour être poussée dans la direction transversale au second axe de pivot.

10. Ensemble selon la revendication 8, dans lequel l'arbre de pivotement est configuré avec une rainure annulaire (126) qui met en prise la clavette de blocage lorsque l'arbre de pivotement est dans la seconde position.

11. Ensemble selon la revendication 10, dans lequel lorsque l'arbre de pivotement est dans la première position, la clavette de blocage et la rainure annulaire ne sont pas mises en prise alors que les languettes d'alignement prévues sur l'arbre de pivotement et l'extrémité de réception de languette d'alignement sont mises en prises et empêchant ainsi le pivotement de l'arbre de pivotement dans le second axe de pivot.
